# EUROPEAN PATENT SPECIFICATION

(11) **EP 2 983 644 B1**
(45) Date of publication and mention of the grant of the patent: **26.01.2022**
(21) Application number: 14716338.0
(22) Date of filing: 10.04.2014
(51) Int. Cl.: A61K 9/28, A61K 9/50, A61K 31/722, A61K 31/66, A61P 3/06, A61K 9/48

(54) **COMPOSITION CONTAINING A POLYAMINOGLYCOSIDE**
ZUSAMMENSETZUNG MIT EINEM POLYAMINOGLYCOSID
COMPOSITION CONTENANT UN POLYAMINOGLYCOSIDE

(30) Priority: 11.04.2013 IT MI20130576
(43) Date of publication of application: 17.02.2016
(73) Proprietor: Labomar S.p.A., 31036 Istrana (TV) (IT)
(72) Inventor: BERTIN, Walter, I-31036 Istrana (TV) (IT); FRATTER, Andrea, I-31036 Istrana (TV) (IT)
(74) Representative: Perani & Partners S.p.A.
(86) International application number: PCT/EP2014/057243
(87) International publication number: WO 2014/167044

(56) References cited:
- WO-A2-2012/153338
- JP-A- 2007 236 222
- KR-B1- 100 750 727
- DATABASE GNPD [Online] MINTEL; March 2012 (2012-03), "Baby Formula 4", XP002719257, Database accession no. 1750738
- DATABASE GNPD [Online] MINTEL; April 2009 (2009-04), "Dietary Supplement for Men over 50", XP002719258, Database accession no. 1081442
- EMILIO ROS: "Intestinal absorption of triglyceride and cholesterol. Dietary and pharmacological inhibition to reduce cardiovascular risk", ATHEROSCLEROSIS, vol. 151, no. 2, 1 August 2000 (2000-08-01), pages 357-379, XP055098203, ISSN: 0021-9150, DOI: 10.1016/S0021-9150(00)00456-1

## Description

The present invention relates to a composition comprising a phosphorylated polymeric aminoglycoside and an organic acid, which comprises in its structure at least one amine function, that at pH between 6.0 and 7.0 is in the form of non- quaternary ammonium salt, a tablet or capsule or granulate comprising said composition and said composition for use in the prevention of intestinal absorption of fatty acids.

Bile salts, or salts of cholic acids are substances in the bile that are secreted in the liver by the hepatocytes and stored in the gall bladder, a gland structurally and functionally connected to the liver. In particular, in the hepatocyte the step of conjugation of cholic acids with the L- Glycine and Taurine occurs, to produce respectively the glycocholic and taurocholic acids. These substances act as surfactants after salification in the enteric environment (duodenum), wherein the pH is greater than or equal to 6. The function of bile is to emulsify fats taken with food in water-soluble micelles, which favor the action of enzymes like lipase in the intestinal environment. The bile helps, especially, the absorption of fatty acids, cholesterol and its esters and glycerides, vitamins and fat-soluble drugs.

The bile include water (85 %), cholesterol (1 %), lecithin, i.e. phosphatidylcholine (1-2 %), bile pigments such as bilirubin (1-2 %) and conjugated bile acids (10 %) as taurocholic, glycocholic, tauroursodeoxycholic, cholic, deoxycholic, ursodeoxycholic acid and / or chenodeoxycholic acid.

The intervention of salified bile acids, i.e. in ionic form (carboxylate), is necessary for the absorption of fats and, in particular, of dietary cholesterol. Bile acids are secreted into the duodenum from the gallbladder through the common bile duct, in response to entry of chyme into the intestine from the gastric tract. The cholic acids, conjugated form with L- glycine and L-taurine, become effective cleaning agents able to emulsify fats, in the form of micelles hydro-dispersible, and then put them in contact with the digestive enzymes present in the brush villi. The cholic acids can exert their detergent - emulsifier action only if salified, and that salification is carried out at a pH greater than their pKa (pH range of 6.5-7.0, that is, that of the first intestinal tract).

Substances, referred to as bile acid sequestrants, are known which are capable of complexing the cholic acids in ionised form, thereby inhibiting their enterohepatic reabsorption. These sequestering agents are basically anion exchange resins comprising a quaternary ammonium salt in the hydrochloride form, such as cholestyramine or colestipol, which bind bile acids in the intestine to form an insoluble complex, which is then excreted via the faeces. However, the therapeutic use of these substances leads to side effects, such as constipation, which can be severe and result in intestinal blockage, discoloration and weakening of teeth as a result of prolonged exposure to the suspension for oral intake, abdominal pain, flatulence, bloating, heartburn and steatorrhea (with loss of fat-soluble vitamins).

The intake of this substance can also lead to intraluminal gallstones, i.e. accumulation of insoluble solids in the intestinal lumen that can give the phenomena of itching and cholecystitis in severe cases. Moreover, the therapy with cholestyramine may cause an increase of the blood triglycerides, of alkaline phosphatase and of transaminase. The use of these ionic resins, finally, is contraindicated in peptic ulcer in the active phase and can increase the risk of gallstones.

A relevant prior art document is JP 2007 236222 A, where a composition containing a salt of chitosan and a slightly soluble organic acid is disclosed as being suitable for the treatment of hypercholesterolemia.

One purpose of the present invention is to provide a composition for use as a medicament in the prevention and treatment of hypercholesterolemia, which does not suffer from the drawbacks of the known art.

One task of the present invention is to provide a composition for the prevention of absorption of fats in the intestine that is devoid of the side effects associated with bile acid sequestrants of the prior art. Another task of the present invention is to provide an efficient, practical and economical method for the prevention of fatty substances absorption in the intestine, by interacting with the process of their emulsification with bile salts.

In accordance with the present invention, these purposes, and others that will become apparent hereinafter, are achieved by A composition comprising:
a) a polymeric aminoglycoside and
b) a phosphorylated organic acid,
   wherein the phosphorylated organic acid b) comprises in its structure at least one amine function, wherein
   - said composition is comprised in a pharmaceutical formulation in the form of tablet, capsule or granules comprising:
      i) an inner core comprising said composition, and
      ii) a coating of said core a) with gastro-resistant film, which coating is freely soluble in the enteric environment and, in general, at pH> 6;
   - at pH between 6.0 and 7.0, when the enteric film ii) is dissolved said phosphorylated organic acid b) is in the form of non-quaternary ammonium salt and at the same pH said polymeric aminoglycoside a) is in polycationic form, whereby a salt is formed between said phosphorylated organic acid in the form of nonquaternary ammonium salt and said polymeric aminoglucoside in polycationic form.

These objects have also been achieved by said composition for use in the prevention of diseases and disorders related to intestinal absorption of fatty acids, in particular of dietary cholesterol, wherein the use comprises the administration of said composition to a subject and its release in the first part of the intestine.

These objects are also achieved via a dietary supplement, medical device, medical food, or medicinal product comprising the composition as described above.

In the context of the present invention, the term "polymeric aminoglycoside" indicates a homo-or heteropolysaccharide or GAG, linear or branched, whose monomeric structure comprising one or more amino groups, that may be free or partially acylated, such as N-acetyl.

In the context of the present invention, "amine function that at pH between 6.0 and 7.0 is in the form of non-quaternary ammonium salt" indicates a primary (- NH₂), secondary (- NHR) or tertiary (NR₂) amino group, that in an aqueous solution at pH between 6.0 and 7.0 can be protonated, completely or at least partially (e.g. more than 20% or more than 50% of the groups), to form the corresponding ammonium ion (- NH₃⁺, - NRH₂⁺, - NR₂H⁺) .

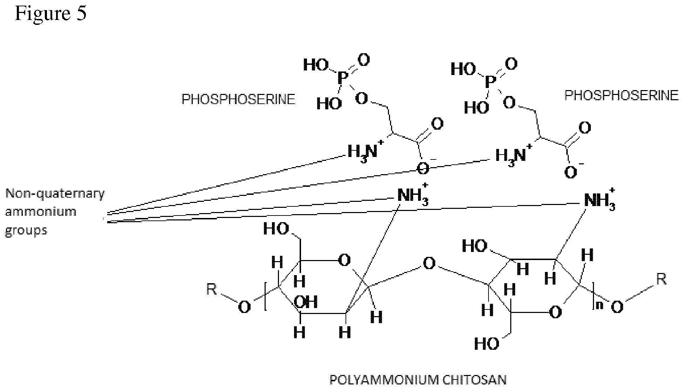

In the context of the present invention, the term "salt" means an ionic compound (Figure 5), that is formed by the exclusively ionic bond between a negatively charged anion (e.g., a carboxylate) and a positively charged cation (e.g., an ammonium), and not a complex or a molecule resulting from the union by means of a covalent bond of two units that carry ionized or ionizable groups.

In the context of the present invention, "phosphorylated organic acid" means a molecule carrying an acidic moiety, such as a carboxylic acid, in a not-derivatized form (i.e. not an ester, an amide, an anhydride or similar acid derivatives), that also carries a phosphate group, for example in the form of of a phosphate ester (-O-PO₃H₂ or -O-PO₃R₂, where R may be the same or different and selected from H or alkyl, aryl, alkenyl or arylalkyl) or N-phosphoramidate (-N-PO₃R₂ where R may be the same or different and selected from H or alkyl, aryl, alkenyl or arylalkyl), wherein said phosphate function emphasizes the acidic character of the substance or is itself the source of acidity. Non limiting examples of such phosphorylated organic acid are phosphoserine, phosphocreatine and similar compounds.

In the context of the present invention, the terms ""polyamino glycosides" and "aminoglycoside polymer" indicate a linear or branched polysaccharide composed of, or comprising, randomly distributed aminosugar units, which may optionally be totally or partiallyN-acetylated. Non-limiting examples of said polymers are chitosan (poly-D-glucosamine) and chitin (poly-N-acetyl-D-glucosamine).

In the present invention, "medical food" indicates a food for special medical purposes, a category recognized by various ministries of health, here between the Italian Ministry of Health and the FDA (5 (b) of the Orphan Drug Act (21 USC 360ee (b) (3)), and intended for patients with particular genetic or metabolic disorders that require treatment or meal replacement in chronic use. They are also defined as "novel foods" and "foods for special medical purposes".

If not otherwise specified, in the present invention the percentages are referred to the weight of a component over the total weight of the composition.
Figure 1 illustrates a clear solution that is obtained salifying 10 grams of tauroursodeoxycholic acid with sodium bicarbonate in saline solution (step 2 of Example 1) .
Figure 2 illustrates the two-phase system obtained by adding Delios^{®} V produced by Cognis (Caprylic / Capric Triglyceride) and cholesterol to a solution mimicking an intestinal solution (pH = 6.8).
Figure 3 (right), the emulsion obtained by adding the solution of Figure 1 to the solution of Figure 2.
Figure 4 illustrates the precipitation of salts of cholic acids (tauroursodeoxycholic acid), obtained by adding the composition according to the invention to the emulsion of Figure 3.
Figure 5 illustrates a non-limiting example of the general formula of a salt between a polymeric aminoglycoside (specifically chitosan) and a phosphorylated organic acid (in particular: phosphoserine) that results from the composition according to the invention.

In one aspect, the present disclosure relates to a composition comprising a polymeric aminoglycoside and a phosphorylated organic acid, which acid comprises in its structure at least one amine function that at pH between 6.0 and 7.0 is in the form of non-quaternary ammonium salt.

It was found that a high complexation of bile salts is obtained by using polymeric aminoglycoside salts including an organic acid carrying both a phosphate group and an amine group that is protonatable at pH such that the polymeric aminoglycoside (as chitosan) is also in the quaternary form (Fig. 5). This evidence makes it possible to reduce the dosage of the active ingredient and can minimize or eliminate the side effects observed with bile acid sequestrants of the prior art. In particular, when chitosan is in salt form, that is water-soluble and gelled, it acts as a soothing and epithelium - regenerating agent. In addition, its antiseptic activity, linked to the presence of an ammonium salt, may be useful in reducing the presence of pathogenic bacterial species. The organic acid that forms part of the composition according to the invention is easily soluble in water (solubility of at least 5 g/100 ml of water) and gives a strong acid reaction, once dissolved in water, with pH of 1% aqueous solution between 2 and 3.

Preferably, in the composition according to the invention the stoichiometric ratio between phosphorylated polymeric aminoglycoside and organic acid is between 1:2.00 and 1:0.75, preferably 1:1.5.

It was also surprisingly found that the organic acid in the composition according to the invention acts synergistically with the polymeric aminoglycoside.

The phosphorylated organic acid of the composition according to the invention can be a derivative, for example, of an alpha-amino acid or of a beta-amino acid, in optically active form (for example non-limiting in enantiomerically pure form) or a racemic form (DL), or another organic acid such as creatine.

Preferably, in the composition according to the invention, the phosphorylated organic acid, comprising at least one amine function that at pH between 6.0 and 7.0 is in the form of non-quaternary ammonium salt, is at least one DL-phosphoserine, D-phosphoserine, L-phosphoserine and phosphocreatine.

For none of these acids an activity is known, in vivo or in vitro, on bile acids or their salts. This action is however particularly advantageous for the purpose of the present invention, even taking into account the fact that there are no reported cases of intolerance or toxicity related to the use of these acids.

More preferably, in the composition according to the invention, the phosphorylated organic acid is DL-phosphoserine.

Preferably, in the composition according to the invention, the polyaminoglicoside is chitosan or another partially or completely N-deacetylated polymer, comprising linear or branched chains of D-glucosamine and N-acetyl-D-glucosamine. More preferably, in the composition according to the invention the polyaminoglicoside is chitosan. The chitosan in the composition according to the invention can be of natural origin (for example by the exoskeleton of crustaceans) or semi-synthetic, it can have different degrees of deacetylation and its molecular weight (Viscosity average molecular weight, Mᵥ) may range from 1,000 to 1,000,000 D, more preferably from 5000 to 500,000 D (the calculation of Mᵥ can be carried out, e.g. as described in: Zamani, A., and Taherzadeh, M. J. Bioresources, 2010, 5, 1554and references cited therein).

Preferably, in the composition according to the invention, the chitosan has a degree of deacetylation equal to or greater than 85%, more preferably greater than 90%, that is not more than 15% or, better, not more than 10% of the amino groups of chitosan are acetylated.

Preferably, in the composition according to the invention, the chitosan has a molecular weight greater than 2000 Dalton and preferably greater than 5000 Dalton. It was found that the use of chitosan of high molecular weight and low degree of acetylation allows an efficient complexation of bile acids in the intestinal tract, also by administering low dosages of the composition according to the invention, provided that it is efficiently converted into cationic form, i.e. protonated, in the intestinal environment with a suitable acid that is capable of introducing additional amino groups.

In another aspect, the present disclosure includes a pharmaceutical formulation in the form of tablet, capsule or granules comprising:
a) an inner core comprising the composition described above; and
b) a coating of said core a) with a gastro-resistant film, which film is instantly soluble at pH> 6 found in the first enteric tract.

It is advantageous that the salification, i.e. the formation of an ionic salt (not a complex or a compound formed through covalent bonds) of polymeric aminoglycoside with the phosphorylated organic acid in the composition of the invention takes place only in the first section of the small intestine, where the acids colonic content in the bile are salified at pH > 6 and around 6.8. The composition as described above is most effective for the complexation of bile salts if the salification of phosphorylated polymeric aminoglycoside by the organic acid occurs only in the intestine and not in the gastric tract, i.e. upstream of the point of secretion of bile salts. To achieve this goal, the tablet, capsule or granules according to the invention allows the release of the composition described above and the formation of the salt of the organic acid and the phosphorylated polymeric aminoglycoside, only in the first intestinal tract, ie, the first contact with the intestinal secretions at pH = 6.8. The so-generated polymeric aminoglycoside in the poly-cationic form binds to salified cholic acids (in anionic form) to form an insoluble complex, which precipitates. The acidic environment which is formed locally by action of the organic acid comprising at least one phosphorylated amino function, which at the intestinal pH is in the form of non-quaternary ammonium salt, further facilitates the breakup of the micelles and precipitation of cholic acids.

The sum of these two phenomena results in the breaking of the lipid emulsion, which normally form at the intestinal level, and the lack of absorption of cholesterol and dietary triglycerides.

It has been found through experimental evidence in vivo that these benefits are achieved by the composition of the invention without the side effects of resins such as cholestyramine and colestipol, especially without causing constipation or the potentially dangerous accumulation in the intestinal lumen of bile salts in solid form (intraluminal cholelithiasis).

Preferably, in the tablet, capsule or granulate according to the invention the content of the composition comprising said phosphorylated polymeric aminoglycoside and said organic acid is between 20 and 80% and preferably between 50 and 70% by weight / total weight of the tablet, capsule or granules.

Preferably, in the formulation according to the invention the stoichiometric ratio polymeric aminoglycoside and phosphorylated organic acid is between 1:2.00 and 1:0.75, more preferably 1:1.5.

Preferably, the enteric coating (b) comprises at least one or a combination of derivatives of methacrylic acid and methyl methacrylate (such as, for non-limiting examples : Eudragit^{®} L100, Eudragit^{®} L12, 5, Eudragit^{®} S100, Eudragit^{®} S12,5), shellac (Shellac^{®}) or methyl cellulose and its derivatives.

Such a coating can be achieved by filming the core a) with a dispersion of the above-indicated polymers, taken individually or in a mixture, for example in a coating pan or by other techniques commonly used and known to those skilled in the art.

In another aspect, the present disclosure relates to a composition as described above for use in the prevention of intestinal absorption of fats, in particular of dietary cholesterol, and diseases and disorders related to it, wherein the use comprises the administration of said composition in the pharmaceutical gastro-resistant oral form and its release in the intestine.

This composition may be useful in the prevention and / or treatment of disorders and diseases such as hypercholesterolemia, obesity, atherosclerosis, metabolic syndrome and hyperlipidemia.

Preferably, the composition according to the invention is for use in the treatment of hypercholesterolemia.

The composition for use according to the present invention is in the form of a tablet or capsule or granulate, coated with a film for release in the small intestine.

The composition for use according to the invention can advantageously be taken concomitantly with meals or immediately after their intake without inducing side effects or intolerance of relief.

Preferably, the composition for use according to the present invention is taken one or more times per day during meals.

In another aspect, the present invention comprises a food supplement, medical device, medical food, or medicinal product comprising the composition as described above. The composition according to the present invention can be administered to a human being or a vertebrate animal other than man.

Preferably, the composition according to the invention can be administered to a mammal, more preferably a human being. An embodiment of the present invention is described below in two examples for illustrative and not limitative scope.

### Example 1.

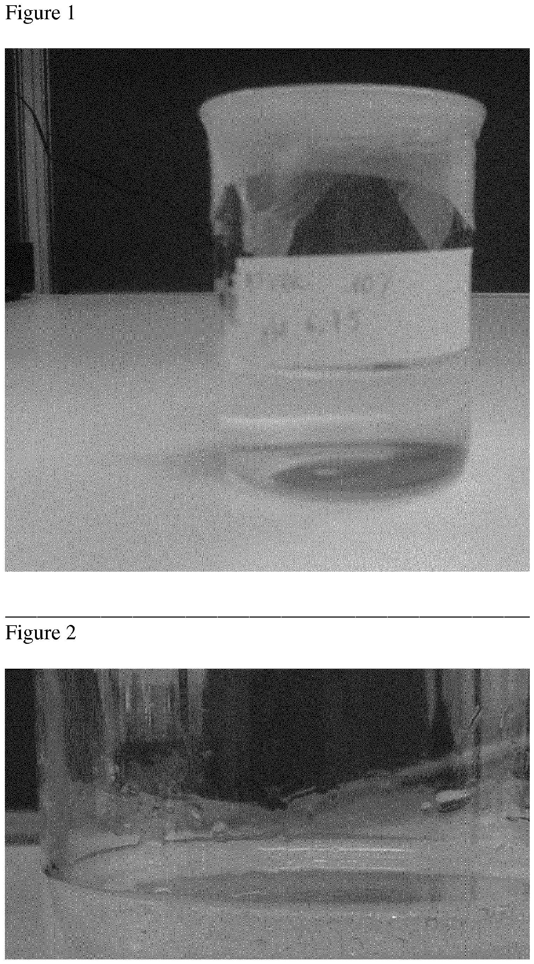

Step 1: a solution mimicking the intestinal secretions with saline duodenal and intestinal buffer solution (phosphate buffer, pH = 6.8) is prepared.
Step 2: in a beaker, a solution mimicking biliary secretion is prepared by dissolving, in saline solution (0.9% NaCl, total volume = 100 ml), tauroursodeoxycholic acid (10 % w / v), a solution of NaOH or Sodium Bicarbonate 10% for neutralization (qs pH = 6.8) to obtain a clear solution (Figure 1).
Step 3: simulation of fat emulsification.

To the "intestinal solution" of step 1, under moderate magnetic stirring and maintained at 37°C, was added a mixture consisting of 3.8 grams of Delios^{®}V, supplied by Cognis (Caprylic / Capric Triglyceride), and 40 mg of cholesterol simulating the intake of fat present in 100 grams of a hamburger bun of the type normally sold in "fast food" stores (3.8 g saturated fat, 36 mg cholesterol per 100 g).

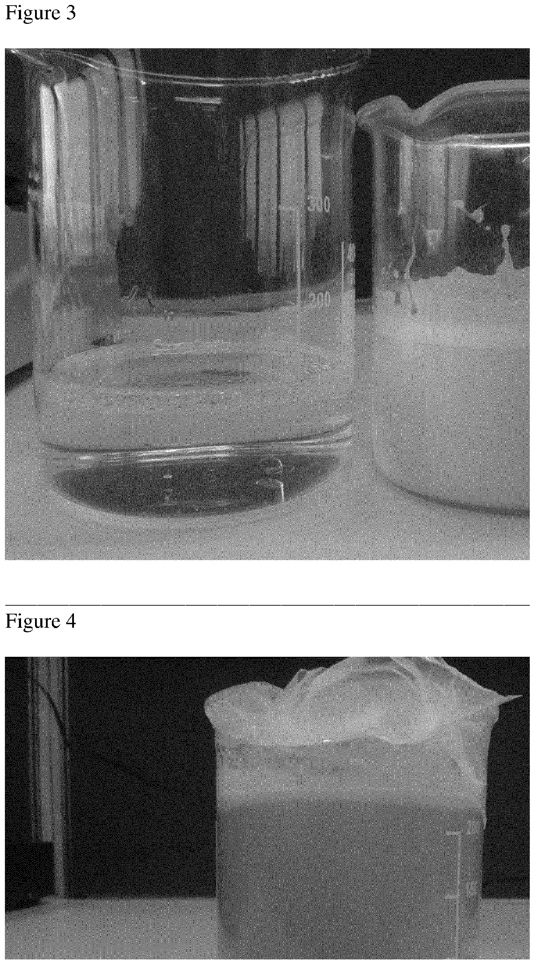

A biphasic system is obtained comprising a clear aqueous solution, in which drops of a second phase are visible that are uniformly dispersed on the bottom and on the surface (Figure 2) of the aqueous phase.
Step 4: under moderate mechanical agitation, the emulsifier phase (bile secretion, step 2) is added to the phase simulating the digestion of fat (fat + intestinal solution, step 3) and to 0.5 grams of phosphatidylcholine (lecithin from sunflower oil). A homogeneous emulsion is obtained with milky appearance constituted by 10 ml of biliary solution (1 gram of cholic acids) and lecithin from sunflower 0.5 g, pH = 6.8 (Figure 3)
Step 5: the composition according to the invention composed of chitosan (0.5 g) and phosphocreatine (0.6 g) (simulation of the tablet core / granulate that dissolves in the intestine) is added to the emulsion of Step 4 to verify the precipitation of bile salts, the acidification of the system and the surface separation of fats. Immediate precipitation of bile salts on the bottom of the solution (Figure 4) is obtained and the observed pH of the solution is 4.00. The identification of the precipitate as salts of cholic acids is performed by methods of using UV spectroscopy after collecting by centrifugation the precipitate, drying it in a ventilated oven and under vacuum and weighing it.

### Example 2.

*In vitro* activity of the composition of the invention.

An initial test is performed adding 5 mg of glycocholic acid (5% w/w solution in water) to 10 ml of simulated gastric or intestinal fluid in the presence of the non-filmed core of a tablet according to the invention (total weight 530 mg, chitosan/phosphoserine content: 250 mg). The amount of free glycocholic acid in the solution is measured by HPLC.

The content of glycocholic acid in the two solutions is summarized in the following table.

**Table 1**

| Conditions | Amount of glycocholic acid in solution (mg) |
|---|---|
| 5 mg of glycocholic acid in gastric fluid-simulating solution + tablet of the invention | 5..3 (± 3) |
| 5 mg of glycocholic acid in intestinal fluid-simulating solution + tablet of the invention | 2.08 |

In the presence of intestinal fluid-simulating solution, the tablet of the invention effectively binds the glycolic acid. This effect is not observed in the presence of gastric fluid.

### Example 3

The binding effect of the composition according to the invention on glycolic acid is measured using simulated intestinal fluid (28 ml), 5% w/w solution of glycolic acid (2 ml, 100 mg of glycolic acid) and a non-filmed core of a tablet according to the invention as above. The tablet core is added to the solution of simulated intestinal fluid and glycocholic acid (30 ml total volume) and the mixture is stirred for 1 h.

The total volume and experimental conditions are selected to replicate the physiological conditions of the intestinal tract.

The experiment is repeated six times.

The content of glycocholic acid in the solution is summarized in the following table.

**Table 2**

| Sample | Amount of glycocholic acid in solution (mg) | % bound glycocholic acid |
|---|---|---|
| 100 mg of glycocholic acid in intestinal fluid-simulating solution + tablet of the invention | 17.4+2.0 | 82.13% |

This finding confirms that the composition according to the present invention is capable of binding cholic acids to a high degree in simulated intestinal fluids.

### Comparative example 1

As a comparative example, the binding capacity of non-salified chitosan towards glycocholic in simulated intestinal solution is measured using simulated intestinal fluid (28 ml), 5% w/w solution of glycolic acid (2 ml, 100 mg of glycolic acid) and a non-filmed tablet comprising chitosan. The tablet is added to the solution of simulated intestinal fluid and glycocholic acid (30 ml total volume) and the mixture is stirred for 1 h.

The experiment is repeated six times.

The content of glycocholic acid in the solution is summarized in the following table.

**Table 3**

| Sample | Amount of glycocholic acid in solution (mg) | % bound glycocholic acid |
|---|---|---|
| 100 mg of glycocholic acid in intestinal fluid-simulating solution + chitosan | 32.6+3.0 | 67.4% |

Non-salified chitosan displays a significantly lower binding capacity with respect to that of the composition according to the present invention.

### Comparative example 2

Using the same experimental conditions as example 2, the binding effect of cholestyramine (Questran^{®} sachet) teoards glycocholic acid in simulated intestinal solution is evaluated (6 experiments, results in Table 4)

**TABLE 4**

| Sample | Amount of glycocholic acid in solution (mg) | % bound glycocholic acid |
|---|---|---|
| 100 mg of glycocholic acid in intestinal fluid-simulating solution + cholestyramine (1 g) | 0.67+0.24 | 99.33% |

Cholestyramine binds substantially all the glycocholic acid initially present in the solution.

Comparing the results of example 3 and comparative examples 1-2, the binding capacity of the composition according to the invention is higher than that of non-salified chitosan, but lower than that of the cationic resin cholestyramine.

### Example 4

To evaluate the mechanism of the binding effect, a series of experiments is carried out using the same experimental conditions of example 3 and dissolving different amounts of glycocholic acid (75 mg, 50 mg, 25 mg and 10 mg).

All tests are repeated in triplicate. The results are summarized in Table 5.

**Table 5**

| Sample | Amount of glycocholic acid in solution (mg) | Initial glycocholic acid in solution (mg) | % bound glycocholic acid |
|---|---|---|---|
| A (3 tests) | 32.6+3.0 | 75 | 81.0 |
| B (3 tests) | 10.43+2.0 | 50 | 79.15 |
| C (3 tests) | 2.31+0.50 | 25 | 90.76 |
| D (3 tests) | 1.90+0.04 | 10 | 84.87 |

The binding capacity of the composition according to the invention appears to vary in a non dose-dependent manner. This may be linked to a prevailing mechanism based on physical binding.

As a further study, at the end of the studies A-D the pH of the solution is modified to 1.5. No significant release of glycocholic acid is observed.

On the basis of these observations and of the fact that non-salified chitosan is less effective that the salified polymer, without wishing to be bound by the theory, it may be inferred that binding of the cholic acids by the compositions of the invention is due to a complex mechanism, wherein both ionic and physical interactions have a relevant role.

## Claims

1. A composition comprising:
a) a polymeric aminoglycoside and
b) a phosphorylated organic acid,
wherein the phosphorylated organic acid b) comprises in its structure at least one amine function, wherein
- said composition is comprised in a pharmaceutical formulation in the form of tablet, capsule or granules comprising:
i) an inner core comprising said composition, and
ii) a coating of said core a) with gastro-resistant film, which coating is freely soluble in the enteric environment and, in general, at pH> 6;
- at pH between 6.0 and 7.0, when the enteric film ii) is dissolved said phosphorylated organic acid b)is in the form of non- quaternary ammonium salt and at the same pH said polymeric aminoglycoside a) is in polycationic form, whereby a salt is formed between said phosphorylated organic acid in the form of nonquatemary ammonium salt and said polymeric aminoglucoside in polycationic form.

2. The composition according to claim 1 wherein the phosphorylated organic acid, comprising at least one amine function that at pH between 6.0 and 7.0 is in the form of non- quaternary ammonium salt, is at least one of DL- phosphoserine, D - phosphoserine, L- phosphoserine and phosphocreatine.

3. The composition according to claim 1 or 2, wherein the polymeric aminoglycoside is chitosan, preferably with a degree of deacetylation greater than or equal to 85 %.

4. The composition according to claim 3, wherein the molecular weight (Mᵥ) of chitosan is greater than 2000 Dalton, preferably greater than 5000 Dalton.

5. The composition according to any one of claims 1-4, wherein the amount of the composition is from 20 to 80 % by weight / total weight of the pharmaceutical formulation, preferably between 50 and 70 %.

6. The composition according to any one of claims 1-5 for use in the prevention of diseases and disorders related to intestinal absorption of fatty acids, in particular of dietary cholesterol, wherein the use comprises the administration of said composition to a subject and its release in the first part of the intestine.

7. The composition for the use according to claim 6 in the prevention and / or treatment of hypercholesterolemia.

8. A dietary supplement, medical device, medical food, or medicinal product comprising the composition according to one of claims 1-5.

## Patentansprüche

1. Zusammensetzung, umfassend:
a) ein polymeres Aminoglykosid und
b) eine phosphorylierte organische Säure,
wobei die phosphorylierte organische Säure b) in ihrer Struktur mindestens eine Aminfunktion umfasst, wobei
- die Zusammensetzung in einer pharmazeutischen Formulierung in Form von Tabletten, Kapseln oder Granulat enthalten ist, die Folgendes umfasst:
i) einen inneren Kern, der die Zusammensetzung umfasst, und
ii) eine Beschichtung des Kerns a) mit einem magensaftresistenten Film, wobei die Beschichtung in der magensaftresistenten Umgebung und im Allgemeinen bei einem pH-Wert > 6 frei löslich ist;
- bei einem pH-Wert zwischen 6,0 und 7,0, wenn der magensaftresistente Film ii) gelöst ist, die phosphorylierte organische Säure b) in Form eines nicht quaternären Ammoniumsalzes vorliegt und bei demselben pH-Wert das polymere Aminoglycosid a) in polykationischer Form vorliegt, wodurch ein Salz zwischen der phosphorylierten organischen Säure in Form eines nicht quaternären Ammoniumsalzes und dem polymeren Aminoglucosid in polykationischer Form gebildet wird.

2. Zusammensetzung nach Anspruch 1, wobei die phosphorylierte organische Säure, die mindestens eine Aminfunktion umfasst, die bei einem pH-Wert zwischen 6,0 und 7,0 in Form eines nicht quaternären Ammoniumsalzes vorliegt, mindestens eines von DL-Phosphoserin, D-Phosphoserin, L-Phosphoserin und Phosphokreatin ist.

3. Zusammensetzung nach Anspruch 1 oder 2, wobei das polymere Aminoglycosid Chitosan ist, vorzugsweise mit einem Deacetylierungsgrad von 85 % oder mehr.

4. Zusammensetzung nach Anspruch 3, wobei das Molekulargewicht (Mᵥ) von Chitosan größer als 2000 Dalton, vorzugsweise größer als 5000 Dalton ist.

5. Zusammensetzung nach einem der Ansprüche 1 bis 4, wobei die Menge der Zusammensetzung 20 bis 80 Gew.- %/ Gesamtgewicht der pharmazeutischen Formulierung, vorzugsweise zwischen 50 und 70 %, beträgt.

6. Zusammensetzung nach einem der Ansprüche 1-5 zur Verwendung bei der Prävention von Krankheiten und Störungen in Verbindung mit der intestinalen Absorption von Fettsäuren, insbesondere von Cholesterin aus der Nahrung, wobei die Verwendung das Verabreichen der Zusammensetzung an eine Person und ihre Freisetzung im ersten Teil des Darms umfasst.

7. Zusammensetzung zur Verwendung nach Anspruch 6 bei der Prävention und / oder Behandlung von Hypercholesterinämie.

8. Nahrungsergänzungsmittel, Medizinprodukt, medizinisches Lebensmittel oder medizinisches Produkt, das die Zusammensetzung nach einem der Ansprüche 1-5 enthält.

## Revendications

1. Composition comprenant :
a) un aminoglycoside polymère et
b) un acide organique phosphorylé,
dans laquelle l'acide organique phosphorylé b) comprend dans sa structure au moins une fonction amine, dans laquelle
- ladite composition est comprise dans une formulation pharmaceutique sous forme de comprimé, de capsule ou de granulés comprenant :
i) un noyau interne comprenant ladite composition, et
ii) un enrobage dudit noyau a) avec un film gastro-résistant, dont l'enrobage est librement soluble dans l'environnement entérique et, en général, à un pH > 6 ;
- à un pH compris entre 6,0 et 7,0, lorsque le film entérique ii) est dissous, ledit acide organique phosphorylé b) se présente sous la forme d'un sel d'ammonium non quaternaire et, au même pH, ledit aminoglycoside polymère a) se présente sous une forme polycationique, un sel est donc formé entre ledit acide organique phosphorylé sous la forme d'un sel d'ammonium non quaternaire et ledit aminoglucoside polymère sous une forme polycationique.

2. Composition selon la revendication 1, dans laquelle l'acide organique phosphorylé, comprenant au moins une fonction amine qui, à un pH compris entre 6,0 et 7,0, est sous la forme d'un sel d'ammonium non quaternaire, est au moins l'un parmi la DL-phosphosérine, la D-phosphosérine, la L-phosphosérine et la phosphocréatine.

3. Composition selon la revendication 1 ou 2, dans laquelle l'aminoglycoside polymère est le chitosane, de préférence avec un degré de désacétylation supérieur ou égal à 85 %.

4. Composition selon la revendication 3, dans laquelle le poids moléculaire (Mᵥ) du chitosane est supérieur à 2000 Dalton, de préférence supérieur à 5000 Dalton.

5. Composition selon l'une quelconque des revendications 1 à 4, dans laquelle la quantité de la composition est comprise entre 20 et 80 % en poids /poids total de la formulation pharmaceutique, de préférence entre 50 et 70 %.

6. Composition selon l'une quelconque des revendications 1 à 5, destinée à l'utilisation dans la prévention de maladies et de troubles liés à l'absorption intestinale d'acides gras, en particulier de cholestérol alimentaire, dans laquelle l'utilisation comprend l'administration de ladite composition à un sujet et sa libération dans la première partie de l'intestin.

7. Composition destinée à l'utilisation selon la revendication 6 dans la prévention et / ou le traitement de l'hypercholestérolémie.

8. Complément alimentaire, dispositif médical, aliment médical ou médicament comprenant la composition selon l'une des revendications 1 à 5.
